(19) Europäisches Patentamt
European Patent Office
Office européen des brevets

(11) Veröffentlichungsnummer: **0 001 384**
**B1**

(12) **EUROPÄISCHE PATENTSCHRIFT**

(45) Veröffentlichungstag der Patentschrift: **27.07.83**

(51) Int. Cl.³: **C 07 C 39/06,** C 07 C 37/16, B 01 J 23/02

(21) Anmeldenummer: **78100440.3**

(22) Anmeldetag: **19.07.78**

(54) **Verfahren zur ortho-Alkylierung von Phenolen.**

(30) Priorität: **07.10.77 DE 2745144**

(43) Veröffentlichungstag der Anmeldung: **18.04.79 Patentblatt 79/8**

(45) Bekanntmachung des Hinweises auf die Patenterteilung: **27.07.83 Patentblatt 83/30**

(84) Benannte Vertragsstaaten: **BE FR GB NL**

(56) Entgegenhaltungen:
**DE-A-2 346 498**
**FR-A-1 063 991**
**FR-A-2 340 296**
**GB-A-972 639**
**US-A-2 678 951**

(73) Patentinhaber: **Rütgerswerke Aktiengesellschaft, Mainzer Landstrasse 217, D-6000 Frankfurt a.Main 1 (DE)**

(72) Erfinder: **Alscher, Arnold, Dr., Dionysius-Platz 3, D-4150 Krefeld 1 (DE)**
Erfinder: **Collin, Gerd, Dr., Hagensallee 56, D-4100 Duisburg-Meiderich (DE)**
Erfinder: **Zander, Maximilian, Dr. Prof., Friedensstrasse 9, D-4620 Castrop-Rauxel (DE)**

**EP 0 001 384 B1**

Anmerkung: Innerhalb von neun Monaten nach der Bekanntmachung des Hinweises auf die Erteilung dès europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur ortho-Alkylierung von Phenolen

Die Erfindung betrifft ein Verfahren zur o-Alkylierung von Phenolen.

In o-Stellung alkylierte Phenolverbindungen sind wichtige Zwischenprodukte und werden z. B. für die Herstellung von Insektiziden, Herbiziden, Antioxidantien, polymeren Polyphenylenäthern und pharmazeutischen Produkten eingesetzt.

Es sind bereits verschiedene Verfahren zur o-Alkylierung von Phenolen mit Alkoholen bekannt. Als Katalysatoren werden hierfür meist Magnesiumoxid und Aluminiumoxid, daneben jedoch auch Mischkatalysatoren beschrieben (K.-D. Bode, in Houben—Weyl, Methoden der organischen Chemie, Bd. VI/1c, S. 1008 ff., G. Thieme Verlag, Stuttgart 1976).

So wird in der GB-PS 1 065 337 die Alkylierung von Phenol mit einem Alkanol beschrieben, wobei die Reaktionskomponenten in der Gasphase über einen gegebenenfalls aktivierten Aluminiumoxidkatalysator geleitet werden. Als Beispiel wird die Umsetzung zu o-Kresol angegeben, das in Ausbeuten von durchschnittlich 27,3 Gewichtsprozent erhalten wird unter gleichzeitiger Bildung von 12,4 Gewichtsprozent 2,6-Dimethylphenol und 5,0 Gewichtsprozent anderer Phenole. (Das Verhältnis der Nebenprodukte zu o-Kresol beträgt also 0,64.)

Nach der DE-PS 1 668 880 wird ein Gemisch von Phenol und Methanol (10 Gewichtsprozent Methanol) bei 250—350° C und einer Flüssigkeitsraumgeschwindigkeit (LHSV) von 0,5 ($h^{-1}$) über einen $\gamma$-Aluminiumoxid-Kontakt mit einer spezifischen Oberfläche (BET) von 240 $m^2/g$ geleitet, wobei ein Produktgemisch von 22% o-Kresol, 5% 2,6-Dimethylphenol und Spuren von Anisol erhalten wurde.

In der GB-PS 1 228 174 wird gefordert, daß das zur o-Methylierung von Phenol verwendete Aluminiumoxid vollständig frei sein soll von insbesondere basischen Verunreinigungen, während geringe Mengen an acider Kontaminierung toleriert werden können: »Die Gegenwart basischer Verunreinigungen im Aluminiumoxid verringert die Aktivität des Katalysators und macht ihn selektiver ... Der Gesamtverlust in der Ausbeute überwiegt jedoch den Vorteil, der sich aus der verbesserten Selektivität ergibt.«

Die GB-PS 1 034 500 beschreibt die Alkylierungsreaktion unter Verwendung von Magnesiumoxid als Katalysator. Aufgrund der relativ geringen Reaktivität des Magnesiumoxid-Katalysators sind jedoch hohe Reaktionstemperaturen von 475—600° C erforderlich, bei denen schon starke Zersetzung der Ausgangsmaterialien, z. B. des Phenols und Methanols, eintritt. Ein weiterer Nachteil liegt in der rasch nachlassenden Aktivität und in der geringen Standzeit dieses Katalysators.

Gemäß der US-PS 2 678 951 werden o-Alkylierungen von 3,5-Dimethylphenol mit Alkanolen in der Gasphase an einem Aluminiumoxidkatalysator durchgeführt, der mit 1—2% Calcium dotiert ist. Das Verfahren zeigt zwar eine gute Alkylierungsrate, jedoch ist die Selektivität zur o-Alkylierung nicht zufriedenstellend.

Die Verwendung von Mischkatalysatoren wie Vanadinoxid/Eisenoxid u. a. bzw. Ceroxid-Manganoxid für die Alkylierung von Phenolen wird in der DE-PS 2 161 252 und in der DE-AS 2 127 083 vorgeschlagen. Diese Katalysatortypen zeigen jedoch eine relativ geringe Reaktivität, so daß insbesondere auch zur Erzeugung von monoalkylierten Produkten ein hoher Überschuß von Methanol eingesetzt werden muß, wodurch die Ausbeute am Monoalkylierungsprodukt stark vermindert wird.

Die aufgeführten Verfahren zeigen somit verschiedene Nachteile, da sie entweder nicht genügend selektiv sind, was zur Bildung von unerwünschten Nebenprodukten führt und/oder nicht reaktiv genug sind, was dann höhere Reaktionstemperaturen, einen Überschuß an Methylierungsmittel bzw. geringe Flüssigkeitsraumgeschwindigkeiten erforderlich macht.

Der Erfindung liegt deshalb die Aufgabe zugrunde, ein Verfahren zur o-Alkylierung von Phenolen in der Gasphase zu entwickeln, das in vorteilhafter Weise Aktivität und Selektivität vereinigt.

Diese Aufgabe wird beim Verfahren zur o-Alkylierung von Phenolen durch Umsetzen eines Phenols mit einem Alkanol in der Gasphase in Gegenwart eines Katalysators, der durch Imprägnieren von Aluminiumoxid mit einer Lösung von Erdalkalisalzen und anschließende Kalzinierung hergestellt ist, erfindungsgemäß dadurch gelöst, daß der Katalysator pro Gramm Aluminiumoxid 0,01 bis 0,1 Millimol Erdalkali enthält und daß die Umsetzung bei Temperaturen im Bereich von 250—400° C und mit einer Flüssigkeitsraumgeschwindigkeit von 0,5—2 $h^{-1}$ durchgeführt wird.

Das zur Herstellung des Katalysators verwendete Aluminiumoxid, vorzugsweise die $\gamma$-Modifikation, kann beispielsweise durch Kalzinieren von $Al_2O_3 \cdot 3\,H_2O$, das durch Ausfällen von Ammoniumsalzen oder Hydrolyse von Aluminiumalkoholat zugänglich ist, hergestellt werden; daneben ist jedoch auch der Einsatz von handelsüblichen aktivierten Tonerdekatalysatoren von hoher Reinheit (Natriumgehalt vorzugsweise unter 0,1%) und von großer spezifischer innerer Oberfläche (spezifische innere Oberfläche (BET) zwischen 200—300 $m^2/g$) möglich. Es ist bekannt, daß reine Tonerde eine inhärente Acidität besitzt, deren Stärke und Verteilung sich nach bekannten Methoden bestimmen läßt (vgl. H. Pines, W. O. Haag, J. Amer. Chem. Soc. 82, 2471 (1960), und A. E. Hirschler, J. of Catal. 2, 428 (1963). Durch die Behandlung mit Erdalkalisalzen und anschließende Kalzinierung wurde die anfänglich etwa 0,2—0,3 Milliäquivalent pro g betragende Acidität der eingesetzten Tonerdekatalysatoren verringert, wobei gleichzeitig auch das Verhältnis der Zahl der starken zu den schwachen Säurestellen vermindert wurde. Jedoch verläuft die Abschwächung der Acidität nicht proportional zur Konzentration des

Erdalkalis im Katalysator.

Die verwendeten Erdalkalisalze, vorzugsweise Bariumsalze, sind beispielsweise Nitrate, Carbonate, Bicarbonate, Oxalate, Acetate. Die Bezeichnung »Erdalkalisalze« soll auch Hydroxide oder Oxide einschließen.

Die nach Imprägnieren und Kalzinieren erreichte Acidität des Katalysators liegt bei etwa 0,05 bis 0,2 Milliäquivalent pro g. Eine Restacidität des Katalysators ist für das erfindungsgemäße Verfahren erforderlich. Eine wesentlich höhere Dotierung mit Erdalkali als 0,1 bis 0,01 Millimol pro g Aluminiumoxid ist unvorteilhaft und führt zur Verringerung der Katalysatoraktivität.

Die mit einer geeigneten, vorzugsweise wäßrigen Lösung der Erdalkalisalze imprägnierten Aluminiumoxid-Katalysatoren werden sorgfältig getrocknet und anschließend bei 450—600°C kalziniert. Die physikalischen Eigenschaften, wie spezifische innere Oberfläche, spezifisches Porenvolumen, Festigkeit und Abrieb werden hierbei nicht verändert. Die Standzeit der Katalysatoren wird durch die angegebene Behandlung mit Erdalkalisalzen nicht verringert und beträgt in Abhängigkeit von den Reaktionsbedingungen zwischen 350 und 1000 Stunden.

Das erfindungsgemäße Verfahren wird bei Temperaturen von 250 bis 400°C, insbesondere von 290—360°C, und bei einer Flüssigkeitsraumgeschwindigkeit (LHSV) von etwa 0,5—2 $h^{-1}$ durchgeführt. Dabei kann sowohl bei normalem als auch bei erhöhtem Druck gearbeitet werden; die Durchführung der Reaktion in Gegenwart von Wasserdampf oder Inertgas ist möglich, jedoch nicht unbedingt erforderlich. Der Katalysator kann in der üblichen Form, z. B. als Festbett oder Fließ- bzw. Wirbelbett angeordnet sein. Das erfindungsgemäße Verfahren eignet sich insbesondere für die Herstellung monoalkylierter Phenole, wobei das molare Verhältnis von Alkylierungsmittel zu phenolischer Verbindung etwa 0,4 bis 2, vorzugsweise 0,4 bis 0,8 beträgt.

Als Ausgangsprodukte werden vorzugsweise Phenole der allgemeinen Formel I

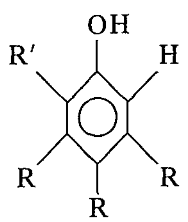

(I)

eingesetzt, worin die Reste R und R′ gleich oder verschieden sein können und ein Wasserstoffatom, eine Alkylgruppe oder auch eine Phenyl- oder eine Benzylgruppe bedeuten. R′ ist vorzugsweise ein Wasserstoffatom.

Die Alkylgruppen können verzweigt oder vorzugsweise geradkettig sein. Sie besitzen vorzugsweise 1—6, insbesondere 1—3 Kohlenstoffatome und sind z. B. Methyl, Äthyl oder Propyl.

Die Phenole der Formel I sind bekannt oder können nach an sich bekannten Methoden hergestellt werden.

Die als Alkylierungsmittel verwendeten Alkanole können verzweigt oder vorzugsweise geradkettig sein. Sie besitzen vorzugsweise 1—6, insbesondere 1 bis 3 Kohlenstoffatome und sind z. B. Methanol, Äthanol oder Propanol.

Für die nachstehenden Ausdrücke gelten folgende Definitionen:

$$\text{Flüssigkeitsraumgeschwindigkeit (LHSV), } (h^{-1}) = \frac{\text{1 Einsatzmat. pro Std.}}{\text{1 Katalysatorschüttvol.}}$$

$$\text{Selektivität, bezogen auf umgesetztes Phenol, \%} = \frac{\text{Gewünschtes Produkt (Mol/h)}}{\text{Phenol im Einsatz (Mol/h)} - \text{nicht umgesetztes Phenol (Mol/h)}}$$

$$\text{Nebenprodukte-Anfall} = \frac{\text{Summe aller flüssigen Nebenprodukte (kg/h) (außer Wasser und Phenol)}}{\text{gewünschtes Produkt (kg/h)}}$$

Prozentangaben beziehen sich auf Gew.-%.

Es wurden drei verschiedene Aluminiumoxid-Katalysatoren A, B, C verwendet; die Zusammensetzung und die physikalischen Daten sind in Tabelle I und II aufgeführt. Als Vergleich werden in Tabelle I und II auch die entsprechenden Daten der unbehandelten Aluminiumoxid-Katalysatoren angegeben.

## Beispiel 1

Durch langsame Zugabe von wäßrigem Ammoniak zu einer auf 90°C erwärmten wäßrigen Lösung von Aluminiumnitrat wurde Aluminiumhydroxid ausgefällt. Der Niederschlag wurde abfiltriert, sorgfältig gewaschen und 30 Std. bei 120°C getrocknet. Anschließend wurde 4 Stunden im Vakuum bei 650°C kalziniert. 1000 g der so hergestellten und zu Preßlingen verarbeiteten aktivierten Tonerde X werden mit einer wäßrigen Lösung von 2,61 g (0,01 Mol) Bariumnitrat imprägniert. Das Volumen der wäßrigen Lösung ist so bemessen, daß es gerade vollständig von dem Tonerdekatalysator aufgenommen wird. Nach 15 Minuten wird der Katalysator bei 110°C getrocknet und 10 Stunden bei 450 bis 500°C unter Stickstoffatmosphäre kalziniert. Man erhält Katalysator A.

In gleicher Weise werden aus den handelsüblichen aktivierten Tonerdekatalysatoren Y und Z die Katalysatoren B und C hergestellt, indem man im Fall von B eine wäßrige Lösung von 7,66 g (0,03 Mol) Bariumacetat und im Fall von C eine wäßrige Lösung von 5,18 g (0,05 Mol) Strontiumacetat verwendet. Die Katalysatorzusammensetzung und die physikalischen Eigenschaften sind in Tabelle I und II angegeben.

Tabelle I

Katalysatorzusammensetzung und physikalische Eigenschaften

| Zusammensetzung und physikalische Eigenschaften | Katalysator X | A | Y | B | Z | C |
|---|---|---|---|---|---|---|
| $Al_2O_3$ (%) | 98,5 | 98,3 | 96 | 95,5 | 98 | 97,5 |
| $SiO_2$ (%) | 0,05 | 0,05 | 1,0 | 1,0 | 0,05 | 0,05 |
| $Na_2O$ (%) | 0,02 | 0,02 | 0,1 | 0,1 | 0,1 | 0,1 |
| $Ba^{2+}$ (%) | | 0,13 | | 0,35 | | |
| $Sr^{2+}$ (%) | | | | | | 0,45 |
| Gewichtsverlust beim Glühen (1 h 900°C) | 1,5 | 1,5 | 2 | 2 | 1,5 | 1,7 |
| Kristallstruktur der krist. Phase | $\gamma$-$Al_2O_3$ geringer Anteil $n$-$Al_2O_3$ | | | $\gamma$-$Al_2O_3$ | | $\gamma$-$Al_2O_3$ geringer Anteil $n$-$Al_2O_3$ |
| Sphärische Form (mm) | Zylindrische Preßlinge, 2×10 | | Extrudat 1,5×4,5 | | Extrudat 1,3×6 | |
| Schüttgewicht (g/cm$^3$) | 0,8 | 0,8 | 0,62 | 0,62 | | |
| Spezifische innere Oberfläche (BET) (m$^2$/g) | 240 | 240 | 250 | 245 | 280 | 270 |
| Porenvolumen (ml/g) | 0,55 | 0,55 | 0,64 | 0,6 | 0,4 | 0,4 |

Tabelle II

Acidität der Katalysatoren, bestimmt durch Titration mit n-Butylamin nach vorangegangenem Glühen bei 500°C

| Indikator | PKa | % $H_2SO_4$ Äquivalent | Milliäquivalente Säure/g Katalysator X | A | Y | B | Z | C |
|---|---|---|---|---|---|---|---|---|
| Phenylazonaphthylamin | +4 | $5\times10^{-5}$ | 0,2 | 0,15 | 0,25 | 0,2 | 0,2 | 0,1 |
| p-Dimethylaminoazobenzol | +3,3 | $3\times10^{-4}$ | 0,15 | 0,05 | 0,25 | 0,2 | 0,2 | 0,1 |
| 4-Phenylazodiphenylamin | +1,5 | $2\times10^{-2}$ | 0,05 | 0,0 | 0,2 | 0,0 | 0,1 | 0,0 |
| Benzalacetophenon | −5,6 | $0,7\times10^{-2}$ | 0,0 | 0,0 | 0,1 | 0,0 | 0,0 | 0,0 |

Beispiel 2

Ein Gemisch aus Methanol und Phenol im molaren Verhältnis 0,5 wird über eine Dosierpumpe bei Normaldruck und mit einer Flüssigkeitsraumgeschwindigkeit von 1 $h^{-1}$ über einen Vorerhitzer durch einen Stahldurchflußreaktor geführt. Das Katalysatorbett hat die Form eines zylindrischen Kreisrings, gebildet durch den Reaktormantel von 30 mm und einer zentral angebrachten Thermoelementschutzröhre von 10 mm. Es wird mit dem mit 0,01 Mol Bariumnitrat vorbehandelten Tonerdekatalysator der Zusammensetzung A beschickt, die Katalysatorfüllung beträgt 200 ml. Der Reaktor wird durch elektrische Widerstandselemente beheizt und auf einer Temperatur von 300° C gehalten, wobei sich am Katalysator eine maximale Temperatur von 320° C einstellt.

Die durchschnittliche Zusammensetzung des Reaktionsprodukts ist in Tabelle III angegeben.

Zum Vergleich werden die unter identischen Reaktionsbedingungen mit dem unvorherbehandelten Tonerdekatalysator X erhaltenen Reaktionsprodukte ebenfalls in Tabelle III ausgeführt.

Tabelle III

| | Katalysator A | A | Vergleichsbeispiel X |
|---|---|---|---|
| Einsatz = Methanol/Phenol im mol. Verhältnis 0,5/1 | | | |
| Hot-spot-Temperatur (°C) | 320 | 320 | 320 |
| Fl.-Raumgeschwindigkeit ($h^{-1}$) | 1 | 1 | 1 |
| Zeit (h) | 10 | 350 | 10 |
| Flüssigprodukt-Ausbeute (%) | 99,4 | 99,2 | 99,3 |
| Produktzusammensetzung (%) | | | |
| Dimethylether und Kohlenwasserstoffe | 0,2 | 0,1 | 0,3 |
| Anisol | 0,3 | 0,4 | 1,2 |
| Phenol | 58,7 | 59,1 | 57,6 |
| o-Kresol | 25,5 | 25,0 | 23,7 |
| m/p-Kresol | 0,4 | 0,4 | 0,8 |
| Dimethylphenol | 5,8 | 6,0 | 6,1 |
| Sonstige Dimethylphenole | 0,8 | 0,8 | 0,9 |
| Trimethylphenole | 0,7 | 0,8 | 1,3 |
| Höhere Phenolhomologe | 0,4 | 0,4 | 1,1 |
| Wasser | 7,1 | 7,0 | 6,9 |
| Summe | 99,9 | 100 | 99,9 |
| Selektivität der o-Kresolbildung (%) | 81,5 | 80,6 | 72,6 |
| Nebenproduktanfall pro t o-Kresol | 0,337 | 0,356 | 0,494 |

Beispiel 3

In der gleichen Weise, wie im Beispiel 2 beschrieben, wird ein Methanol-Phenol-Gemisch vom molaren Verhältnis 0,4 an dem mit 0,01 Mol Bariumnitrat vorbehandelten $\gamma$-Aluminiumoxid-Katalysator A umgesetzt. Der Reaktor wird auf etwa 320° C geheizt, wobei sich am Katalysator eine maximale Temperatur von 330° C einstellt. Die Flüssigkeitsraumgeschwindigkeit beträgt 1,5 $h^{-1}$.

Zum Vergleich wird unter identischen Reaktionsbedingungen die Umsetzung auch an dem unvorbehandelten Tonerdekatalysator X durchgeführt.

Die Reaktionsprodukte weisen folgende durchschnittliche Zusammensetzungen auf (Tabelle IV):

Tabelle IV

| | Katalysator A | A | A | Vergleichsbeispiel X |
|---|---|---|---|---|
| Einsatz = Methanol/Phenol im mol. Verhältnis 0,4/1 | | | | |
| Hot-spot-Temperatur (°C) | 330 | 330 | 330 | 330 |
| Fl.-Raumgeschwindigkeit ($h^{-1}$) | 1,5 | 1,5 | 1,5 | 1,5 |
| Zeit (h) | 10 | 100 | 300 | 10 |
| Flüssigprodukt-Ausbeute (%) | 99,5 | 99,5 | 99,4 | 99,5 |
| Produktzusammensetzung (%) | | | | |
| Dimethylether und Kohlenwasserstoffe | Spuren | Spuren | 0,1 | 0,1 |
| Anisol | 0,4 | 0,1 | 0,5 | 1,0 |
| Phenol | 63,3 | 61,6 | 63,5 | 62,7 |
| o-Kresol | 23,1 | 24,4 | 22,7 | 22,0 |
| m/p-Kresol | 0,4 | 0,7 | 0,4 | 0,5 |
| Dimethylphenol | 4,7 | 5,3 | 4,8 | 5,0 |
| Sonstige Dimethylphenole | 0,7 | 1,5 | 0,8 | 1,2 |
| Trimethylphenole | 0,7 | – | 0,6 | 0,8 |
| Höhere Phenolhomologe | 0,2 | – | 0,2 | 0,3 |
| Wasser | 6,5 | 6,5 | 6,4 | 6,3 |
| Summe | 100 | 100,1 | 100 | 99,9 |
| Selektivität der o-Kresolbildung (%) | 80,0 | 79,2 | 78,9 | 74,4 |
| Nebenproduktanfall pro t o-Kresol | 0,307 | 0,312 | 0,326 | 0,405 |

Beispiel 4

Ein Gemisch aus Methanol und 3,5-Dimethylphenol im molaren Verhältnis 0,5 : 1 wird bei einer Flüssigkeitsraumgeschwindigkeit von 1 $h^{-1}$ in gleicher Weise, wie im Beispiel 2 beschrieben, an dem mit 0,05 Mol Strontiumacetat vorbehandelten Katalysator C umgesetzt.

Die durchschnittliche Zusammensetzung der Reaktionsprodukte sowie die Ergebnisse der unter identischen Bedingungen am unvorbehandelten Tonerdekatalysator Z durchgeführten Umsetzung sind in der nachfolgenden Tabelle V angegeben.

Tabelle V

| | Katalysator C | Vergleichsbeispiel Z |
|---|---|---|
| Einsatz = Methanol/3,5-Dimethylphenol im mol. Verhältnis 0,5/1 | | |
| Hot-spot-Temperatur (°C) | 330 | 330 |
| Fl.-Raumgeschwindigkeit ($h^{-1}$) | 1,3 | 1,3 |
| Zeit (h) | 8 | 8 |
| Flüssigprodukt-Ausbeute (%) | 99,2 | 99,3 |
| Produktzusammensetzung (%) | | |
| Niedere Phenolhomologe, Ether und Kohlenwasserstoffe | 2,6 | 2,8 |
| 3,5-Dimethylphenol | 56,8 | 55,9 |
| 2,3,5-Trimethylphenol | 27,1 | 25,9 |
| 2,3,5,6-Tetramethylphenol | 5,8 | 6,4 |
| Sonstige Alkylphenole | 0,4 | 1,5 |
| Wasser | 7,3 | 7,4 |
| Summe | 100 | 99,9 |
| Selektivität der Trimethylphenol-Bildung (%) | 75,2 | 70,3 |
| Nebenproduktanfall pro t 2,3,5-Trimethylphenol | 0,32 | 0,413 |

Beispiel 5

In der gleichen Weise, wie im Beispiel 2 beschrieben, wird ein Ethanol-Phenol-Gemisch im molaren Verhältnis 0,4 mit einem Wassergehalt von etwa 3% an dem mit Bariumnitrat vorbehandelten Tonerdekatalysator der Zusammensetzung B umgesetzt.

Die Flüssigkeitsraumgeschwindigkeit beträgt 1 $h^{-1}$, die maximale Temperatur am Katalysator wird auf 325° C eingestellt.

Das Reaktionsprodukt weist die in Tabelle VI angegebene durchschnittliche Zusammensetzung auf. Zum Vergleich werden ebenfalls die unter identischen Reaktionsbedingungen an dem unvorbehandelten Tonerdekatalysator Y erhaltenen Ergebnisse aufgeführt.

Tabelle VI

| | Katalysator B | Vergleichsbeispiel Y |
|---|---|---|
| Einsatz = Ethanol/Phenol/Wasser im mol. Verhältnis 0,4/1/0,17 | | |
| Hot-spot-Temperatur (°C) | 325 | 325 |
| Fl.-Raumgeschwindigkeit ($h^{-1}$) | 1 | 1 |
| Zeit (h) | 8 | 8 |
| Flüssigprodukt-Ausbeute (%) | 99,9 | 99,8 |
| Produktzusammensetzung (%) | | |
| Diethylether und Kohlenwasserstoffe | Spuren | Spuren |
| Phenetol | Spuren | 0,1 |
| Phenol | 58,7 | 60,3 |
| o-Ethylphenol | 24,5 | 22,3 |
| m/p-Ethylphenol | 2,3 | 2,0 |
| Diethylphenole | 3,4 | 3,5 |
| Höhere Phenolhomologe | 2,3 | 2,4 |
| Wasser | 8,8 | 9,4 |
| Summe | 100 | 100 |
| Selektivität der Ethylphenolbildung (%) | 85,1 | 82,8 |
| Nebenprodukt pro t Ethylphenol | 0,338 | 0,359 |

**Patentansprüche**

1. Verfahren zur ortho-Alkylierung von Phenolen durch Umsetzen eines Phenols mit einem Alkanol in der Gasphase in Gegenwart eines Katalysators, der durch Imprägnieren von Aluminiumoxid mit einer Lösung von Erdalkalisalzen und anschließende Kalzinierung hergestellt ist, dadurch gekennzeichnet, daß der Katalysator pro Gramm Aluminiumoxid 0,01 bis 0,1 Millimol Erdalkali enthält und daß die Umsetzung bei Temperaturen im Bereich von 250—400° C und mit einer Flüssigkeitsraumgeschwindigkeit von 0,5—2 $h^{-1}$ durchgeführt wird.

2. Verfahren nach Anspruch 1, dadurch gekennzeichnet, daß man als Ausgangsmaterial Phenole der allgemeinen Formel I

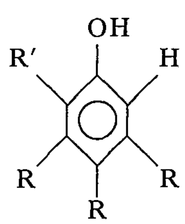

(I)

einsetzt, worin die Reste R und R′ gleich oder verschieden sein können und ein Wasserstoffatom, eine Alkylgruppe oder auch eine Phenyl- oder eine Benzylgruppe bedeuten.

3. Verfahren nach Ansprüchen 1 oder 2, dadurch gekennzeichnet, daß man als Alkylierungsmittel ein Alkanol mit 1—6 Kohlenstoffatomen verwendet.

## Claims

1. A process for the ortho-alkylation of phenols by reaction of a phenol with an alkanol in the gaseous phase and in the presence of a catalyst which is obtained by impregnation of an aluminium oxid with a solution of an alkaline earth metal salt and subsequent calcination characterized in that the catalyst contains 0,01 to 0,1 millimole of alkaline earth metal per gram of aluminium oxid and that the reaction is conducted at temperatures in the range of 250—400° C and at a liquid hourly space velocity in the range of 0,5—2 $h^{-1}$.

2. A process according to claim 1, characterized in that phenols of the general formula I

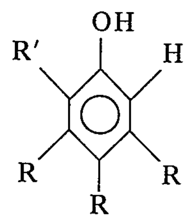

(I)

are used as starting material, wherein the radicals R and R′ may be the same or different and signify a hydrogen atom, an alkyl group, a phenyl group or a benzyl group.

3. A process of the claim 1 or 2 characterized in that as alkylation agent an alkanol with 1—6 carbon atoms is used.

## Revendications

1. Procédé pour l'ortho-alcylation des phénols par réaction d'un phénol avec un alcanol en phase gazeuse en présence d'un catalyseur qui est préparé par imprégnation d'alumine à l'aide d'une solution de sels alcalino-terreux suivie d'une calcination, caractérisé en ce que le catalyseur contient de 0,01 à 0,1 millimole d'élément alcalino-terreux par gramme d'alumine et en ce que la réaction est effectuée à des températures dans l'intervalle de 250 à 400° C et avec une vitesse spatiale horaire à l'état liquide de 0,5 à 2 $h^{-1}$.

2. Procédé selon la revendication 1, caractérisé en ce que l'on utilise en tant que produit de départ des phénols de formule générale I:

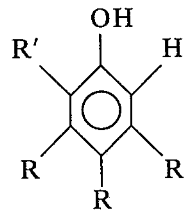

(I)

dans laquelle les symboles R et R′, qui peuvent être identiques ou différents, représentent un atome d'hydrogène, un groupe alcyle ou encore un groupe phényle ou benzyle.

3. Procédé selon la revendication 1 ou 2, caractérisé en ce que l'on utilise en tant qu'agent alcylant un alcanol contenant de 1 à 6 atomes de carbone.